# EUROPEAN PATENT SPECIFICATION

(11) **EP 2 192 935 B1**
(45) Date of publication and mention of the grant of the patent: **04.03.2020**
(21) Application number: 08802693.5
(22) Date of filing: 29.09.2008
(51) Int. Cl.: A61M 5/145, A61M 5/142

(54) **CARTRIDGE ADAPTER FOR USE IN AN INFUSION SYSTEM**
KARTUSCHENADAPTER ZUR VERWENDUNG IN EINEM INFUSIONSSYSTEM
ADAPTATEUR DE CARTOUCHE DESTINÉ À ÊTRE UTILISÉ DANS UN SYSTÈME DE PERFUSION

(30) Priority: 01.10.2007 US 976500 P
(43) Date of publication of application: 09.06.2010
(73) Proprietor: Roche Diabetes Care GmbH, 68305 Mannheim (DE); F. Hoffmann-La Roche AG, 4070 Basel (CH)
(72) Inventor: KRAFT, Torsten, 4500 Solothurn (CH); STOLLER, Hanspeter, 3013 Bern (CH); SCHEURER, Simon, 3006 Bern (CH); THALMANN, Christian, 6365 Kehrsiten (CH)
(74) Representative: Rentsch Partner AG
(86) International application number: PCT/EP2008/008256
(87) International publication number: WO 2009/043564

(56) References cited:
- WO-A2-2005/000378
- US-A- 5 520 653
- US-A- 5 776 116
- US-A1- 2002 173 748
- US-A1- 2003 130 618
- US-A1- 2007 167 912
- US-B1- 6 277 095

## Description

### RELATED APPLICATION

This application claims priority to US Provisional application No 60/976500 filed on October 1, 2007.

### TECHNICAL FIELD

The present invention generally relates to an infusion pump for dispensing medication. More specifically it relates to an adapter that can be attached to a fluid cartridge for dispensing medication from the infusion pump.

### BACKGROUND

Medical devices that pump medication into an individual is known and commonly used in the medical industry. Typically the medication that is delivered from such medical devices depends on the medical condition that is sought to be treated. For example, it is getting increasingly common to deliver insulin using an insulin pump to treat a diabetic patient.

Typically, the medical pump devices use a reservoir or a cartridge that contains the medicine to be delivered. Depending on the size of the cartridge that needs to be used may determine the overall size of the medical pump system. Since manufacturing of a medical pump device is expensive, manufactures typically decide which size cartridge they are going to use and design their medical pump system based on such consideration. This limits the ability of patients receiving treatments to only use medication that is pre-determined by the manufacture of the medical pump system.

Therefore, there is a need in the medical industry to have the flexibility to use any sized cartridge in any medical pump device. In other words there is a need to reduce the interdependency of the size of the cartridge and medical pump device of choice.

US 2003/0130618 A1 describes a loading mechanism for an infusion pump system. The mechanism comprises a medication reservoir with a plunger and a threaded plunger rod offset from the axis of the reservoir, an adapter for aligning and offsetting the reservoir, and a hub with a handle. The infusion pump comprises a drive screw that is offset from the axis of a pump barrel. The pump barrel receives the assembly comprising hub, adapter, reservoir, plunger and plunger rod. By rotating the hub by 90 degrees, the hub is coupled to the pump housing and the threaded plunger rod is engaged with the drive screw.

Documents US 2007/167912 A1, WO 2005/000378 A2 and US 2002/173748 A1 describe further prior-art infusion pumps with advantageous replaceable cartridges.

### SUMMARY

It is against the above background that the present invention proves certain unobvious advantages and advancements over the prior art. The scope of the invention is defined in the independent claim. Further embodiments are defined in the dependent claims.

The present invention comprises an infusion system for pumping fluid into a body of a user, the infusion system has an a infusion pump with a housing, the housing includes a cavity. The cavity is designed to receive a fluid storing means, such that the a fluid storing means can be removably inserted into the housing. The fluid storing means comprises a cartridge and an adapter, such that the adapter removably encases the cartridge. In addition, the infusion system also has a cap that is attached to the housing of the infusion system for holding the fluid storing means in place inside the housing of the infusion pump.

The adapter comprises a hollow housing such that the cartridge can be snapped into the interior of the housing of the adapter. The adapter may also comprises a canula or a needle that can pierce the septum of the cartridge when the adapter encases the cartridge. The adapter is designed such that it can receive different size cartridge, but can fit into the infusion pump.

The adapter in addition to the needle also includes the connection means to connect an infusion set. The connection means is the industry standard luer connection or as proprietary connection to the infusion set.

In yet another embodiment of the present invention comprises an infusion system for pumping fluid into a body of a user, the infusion system has an infusion pump with a housing, the housing includes a cavity. A fluid storing means is removably inserted into the cavity of the housing. The fluid storing means comprises a cartridge and an adapter, such that the adapter removably encases the cartridge. In addition, the infusion system also has a cap that is attached to the housing of the infusion system for holding the fluid storing means in place inside the housing of the infusion pump.

The adapter comprises a housing that is formed of two parts. One part of the housing is placed into the cavity of the infusion pump. This part of the housing may be removably or fixedly attached into the cavity. The second part comprising the canula and the connecting means is coupled to the first part after the cartridge is inserted into the adapter.

In yet another embodiment of the present invention the adapter is designed such that it is possible to use a kind of pen cartridge which has a code cap.

In yet another embodiment, the adapter can be designed such that it works as a spring or a shock absorbing element to cover the tolerances between the cartridge and the housing of the insulin pump. In order to achieve that the hollow interior of the adapter may be provided with a spring mechanism or any shock absorbing material.

In yet another embodiment where the adapter is formed of two parts, the shock absorbing element is provided in the part that is attached to the pump housing.

In yet another embodiment, the adapter may be provided with a valve between the canula and the connecting means to prevent free flow of the medication from the cartridge into the infusion set when the plunger is not connected to the drive mechanism.

In yet another embodiment, the adapter is provided with a mechanism to transfer torque from the adapter onto the cartridge. For example, such mechanism could include a plurality of ribs provided in the interior of the housing of the adapter.

These and other features and advantages of the present invention will be more fully understood from the following detailed description of the invention taken together with the accompanying claims. It is noted that the scope of the claims is definitely by the recitations therein and not by the specific discussion of the features and advantages set forth in the present description.

### BRIEF DESCRIPTION OF THE DRAWINGS

The following detailed description of the embodiments of the present invention can be best understood when read in conjunction with the following drawings, where like structure is indicated with like reference numerals and in which:
FIGURE 1 is an exploded view of the infusion delivery system in accordance with the teachings of the present invention;
FIGURE 2 is a perspective view of the cartridge and the adapter in the infusion pump of Fig 1;
FIGURE 3 is a perspective view of the cartridge and the adapter in accordance to the teachings of the current invention;
FIGURE 4 is a perspective view of the adapter in accordance with the teachings of the current invention;
FIGURE 5 is a cross-sectional view of the adapter along lines A-A in Figure 4;
FIGURE 6 is an end view of the adapter along line B in Figure 4; and
FIGURE 7 is a perspective view of the cartridge and the two part adapter housing in the infusion pump of Fig 1.

Skilled artisans appreciate that elements in the figures are illustrated for simplicity and clarity and have not necessarily been drawn to scale. For example, the dimensions of some of the elements in the figure may be exaggerated relative to other elements to help improve understanding of the embodiment(s) of the present invention.

### DETAILED DESCRIPTION

The following description of the preferred embodiment is merely exemplary in nature and is in no way intended to limit the invention or its application or uses.

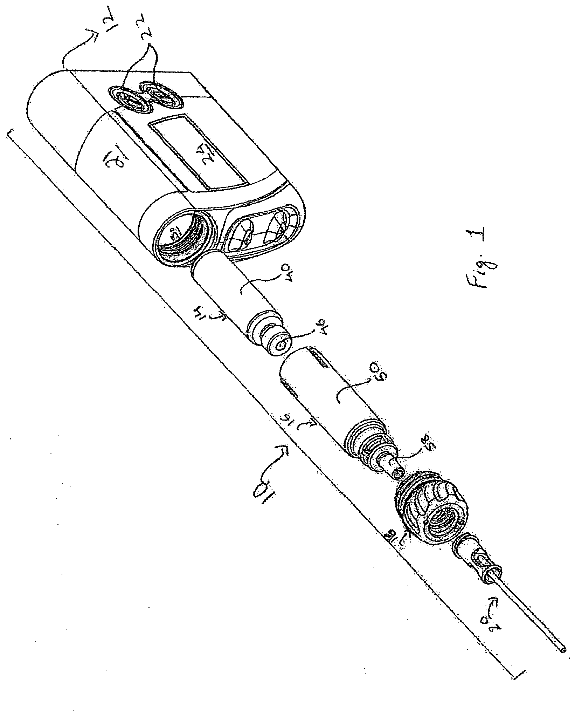

Referring in particular to Figure 1, an infusion delivery system is generally represented by reference numeral 10. As can be seen in the drawing, the infusion delivery system comprises an infusion pump 12, a fluid storing means 15, a cap 18 to hold the fluid storing means 15 in place and an infusion set (not completely shown) 20 that is connected to the fluid cartridge through the cap 18. The infusion pump shown in the drawings is an insulin pump 12 such as the one sold by Disetronic Medical System under the name Accu-Chek Spirit ®. Although an insulin pump is shown, it must be understood that this invention is not limited to insulin pumps but to any pump that can be used to deliver medication.

The infusion pump 12 comprises a housing 21 that has at least one control 22 to control the infusion of medication from fluid storing means 15. The infusion pump 12 also includes a display 24 to display information relevant to the operation of the infusion pump 12. Alternatively, the infusion pump 12 may have no display and only controls or be void of both display and controls. Although not shown in the drawings, the infusion pump 12 may have additional controls or buttons to effectively operate the infusion pump 12. Although not shown in the drawings the infusion pump 12 may be controlled remotely to dispense medication using a remote control device such as a smart phone, a PDA or any other mobile devices. Although not specifically mentioned, the infusion pump 12 may be a one time use pump such that after the dispensing of the medication the pump is disposed.

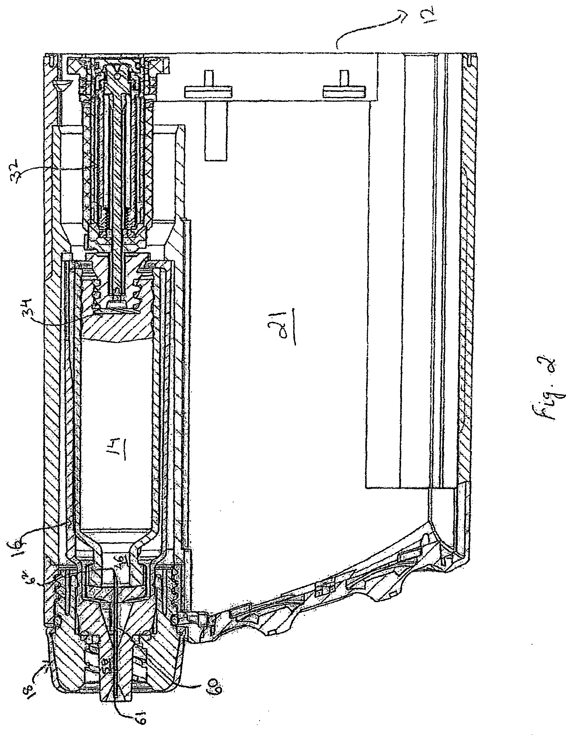

With reference to Figures 1 and 2, the fluid storing means 15 comprises a cartridge 14 and an adapter 16. The housing 21 of the infusion pump 12 has a cavity 30 for receiving the fluid storing means 15. The fluid storing means 15 can be removably inserted into the cavity such that after the medication is over, a new fluid storing means 15 can be inserted into the cavity 30. The fluid storing means 15 when inserted into the opening 30 cooperates with a drive system 32 that advances a plunger 34 so as to dispense medication thereform. The drive system 32 and the plunger 34 are well known in the art and are not explained in detail. In operation, when the infusion pump 12 is given the required commands/instructions, the drive system 32 moves the plunger such that the medication is dispensed from the cartridge 14 through the infusion set 20.

The cartridge 14 contains a medication (not shown) that needs to be dispensed. For example, if the infusion pump 12 is an insulin pump, then the cartridge 14 contains insulin. The medication may be prefilled into the cartridge such that when the medication is empty, the user throws the cartridge away. One such example of a pre-filled cartridge 14 is sold by Novo Nordisk A/S under the name Novolog® Alternatively, the cartridge 14 may be designed such as the user fills the required medication as needed from a vial provided by the manufacturer of the appropriate medication . As seen in the drawing, the cartridge 14 contains a housing 40 having a first end 42 and a second end 44. Depending on the medication used and its expected shelf life, the housing 40 can be made of plastic, glass or any other suitable material. In one embodiment, when the cartridge 14 is inserted into the cavity 30 the second end 44 cooperates with the drive system 32 of the infusion pump 12. The first end 42 of the cartridge 14 comprises a sealable septum 46 into which a hollow needle from the adapter 16 can be removably inserted (as will be explained in detail). Alternatively, in another embodiment, the cartridge 14 and the adapter 16 are coupled together and then inserted as a unit into the cavity 30 of the infusion pump 12. In this embodiment, the distal end 44 of the cartridge 14 couples with the drive unit 32 such that medication from the cartridge 14 can be infused in a controlled manner.

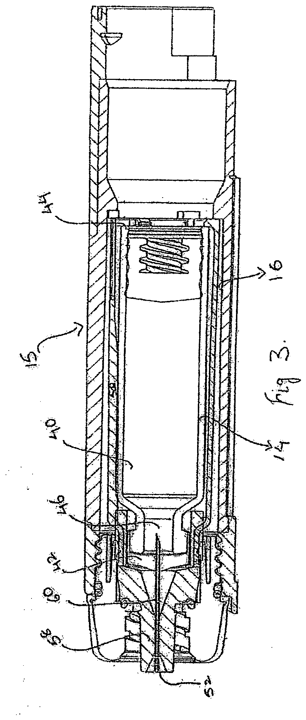

With reference to Figures 2, 3 and 4, the adapter 16 comprises a housing 50 having a first end 52 and a second end 54. The housing 50 of the adapter 16 has a hollow interior 56 such that the cartridge 14 can be removably received in the interior 56. In such an arrangement, the adapter 16 encases the cartridge 14. The shape of the housing 50 is such that the adapter 16 can easily fit into the cavity 30 of the infusion system 12. In addition, the shape is compatible with the outer shape of the cartridge housing 40. As shown, the first end 52 of the adapter is open and the cartridge 14 can be inserted into the adapter 16 through this end. In the embodiment where the cartridge 14 is first inserted into the infusion pump 12, the adapter 16 is slidably engaged on top of the cartridge 14 such that it covers the cartridge 14. In the embodiment where the cartridge 14 and the adapter 16 are inserted as a unit into the infusion pump 14, the cartridge 14 slides into the hollow interior 50 of the adapter 16 thereby forming the fluid storing means that is then inserted into the cavity 30 of the housing 21.

With continued reference to the drawings, the second end 54 of the housing 50 comprises a connection means 58 for connecting the fluid storing means 15 to the infusion set 20. The connection means 58 is a standard luer connection or a proprietary connection to connect the infusion set 20 to the infusion pump 12. The second end 54 of the adapter 16 also includes an integrated canula 60. As shown the canula 60 at one end 61 is fixedly connected to the interior of the adapter housing 50. The canula 60 at the opposite end has a sharp tip 62 that extends inside the body of the housing 50 of the adapter 16. When the cartridge 14 is inserted inside the adapter 16 or when the adapter 16 slides over the cartridge 14, the canula 60 pierces the septum 46 of the cartridge 14 such that it is in contact with the medication inside the cartridge 14. The canula 60 is hollow such that medication can flow into the infusion set 20. The canula 60 can be formed from plastic or other materials such as steel.

Although not specifically shown in the drawing, the adapter 16 has attaching means for holding the cartridge 14 in place. Such attaching means may be provided at the first end 52 or the second end 54 of the adapter housing 50. The attaching means can be provided in the interior of the adapter housing 50 or can be provided at any other suitable place. For example, in order to ensure that the adapter 16 can receive different cartridge sizes into the hollow interior 56 , it is possible to design the interior housing 50 to have some spring means such that the cartridge 14 is snapped into the adapter 16. The attaching means to attach or snap fit the adapter 16 may include groves, ridges, recess or any other mechanism that will help a tight fit between the adapter 16 and the cartridge 14.

In operation once the adapter 16 and the cartridge 14 are connected with each other it may not be possible to separate them and reuse one or the other. In other words once the medication is over from the cartridge 14, they will be removed as a unit from the infusion pump and disposed as a unit. Alternatively, it may be possible to separate the adapter 16 from the cartridge 14 and dispose them off separately.

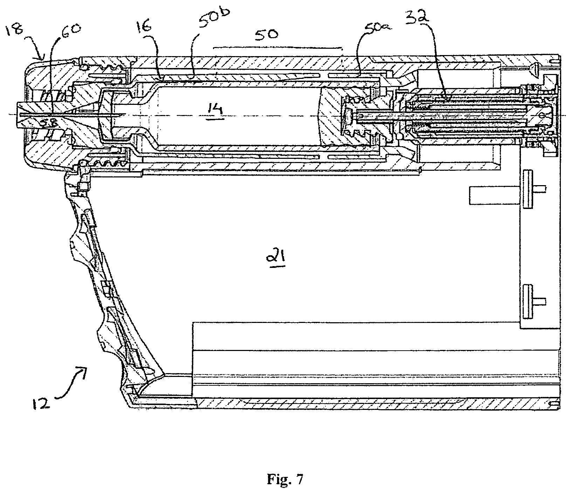

In another embodiment as shown in Fig. 7, the adapter 16 comprises a housing 50 that is formed of two parts 50a and 50b. One part 50a of the housing is placed into the cavity 30 of the infusion pump 12. This part of the housing may be removably or fixedly attached into the cavity 30 and around the plunger of the infusion pump. The second part 50b comprising the canula 60 and the connecting means 58 is coupled or mated to the first part 50a of the housing after the cartridge 14 is inserted into the adapter 16. The second part 50b can be coupled to the first part 50a by different method such as just placing the second part 50b on top of the first part 50a. alternatively, the first part 50a or the second part 50b can be connected by snapping the two parts together or fastening the two parts together.

In this embodiment, where the adapter housing 50 is formed of two parts 50a and 50b, it is possible to transfer some of the functionalities from the housing of the adapter to the part 50a that is attached to the cavity of the pump housing. For example, the part 50a may be provided with a spring or a shock absorbing material to cover the tolerances between the adapter and the pump housing. In addition, the part 50a may be coded or provided with a coding mechanism such that it detects correct attachment of the cartridge and/or the adapter into the housing of the pump.

In yet another embodiment of the present invention the adapter is designed such that it is possible to use a kind of pen cartridge which has a code cap.

In yet another embodiment, the adapter 16 can be designed such that it works as a spring or a shock absorbing element to cover the tolerances between the cartridge 14 and the housing 21 of the infusion pump 12. In order to achieve that the hollow interior 56 of the adapter 16 may be provided with a spring mechanism or any shock absorbing material.

In yet another embodiment, the adapter 16 may be provided with a valve between the canula 60 and the connecting means 58 to prevent free flow of the medication into the infusion set if the plunger is not connected to the drive mechanism.

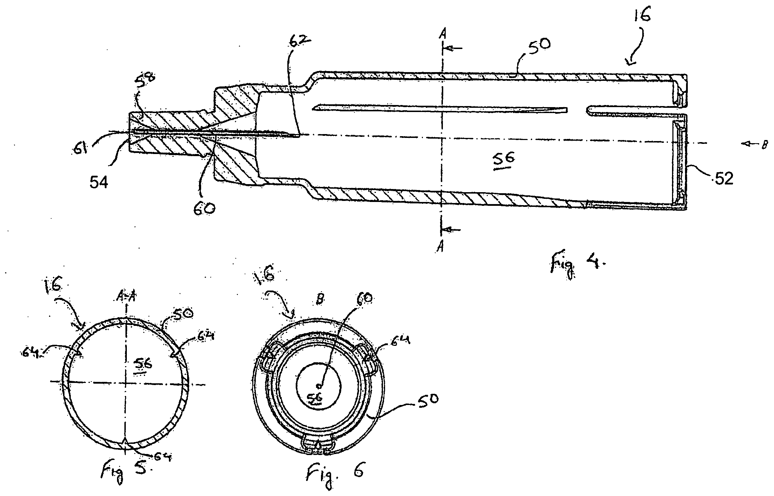

In yet another embodiment, the adapter 16 is provided with a mechanism to transfer torque from the adapter 16 onto the cartridge 14. For example, such mechanism could include a plurality of ribs 64 (as shown in Figure 5 and 6) provided in the hollow interior 56 of the adapter housing 50 .

The cap 18 is coupled to the infusion pump housing 21 after the fluid storage means 15 is inserted into the cavity 30. The cap 18 serves to retain the cartridge 14 in the infusion pump 12. In addition, the cap 18 also helps perform a number of functions for the infusion pump 12 to operate. For example, attachment of the cap 18 may lock the cartridge 14 in the pump housing 21 and ceasing or preventing the dispensation of medication if the cap 18 is not properly engaged with the insulin pump 12. In addition, the cap may serve to water proof the infusion pump 12. In cases where the infusion pump 12 is a one time disposable pump the cap may lock the fluid storage means such that it can not be reused by the user.

To operate the infusion delivery system, the user inserts the fluid storing means 15 into the cavity 30 such that the canula 60 integrated into the adapter 16 pierces the septum 46 of the cartridge 14 to create a fluidic path. The cap 18 is then coupled to the housing 21 such that the fluid storing means 15 is held in place inside the cavity 30, thereby engaging the drive system 32. The connection means 58 on the adapter 16 is then connected to the infusion set 20. The user then initiates commands/instructions either using the controls on the infusion pump 12 or a remote device to pump the medication from the cartridge 14 into the body of the user.

It is noted that terms like "preferably", "commonly", and "typically" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

For the purposes of describing and defining the present invention it is noted that the term "substantially" is utilized herein to represent the inherent degree of uncertainty that may be attributed to any quantitative comparison, value, measurement, or other representation. The term "substantially" is also utilized herein to represent the degree by which a quantitative representation may vary from a stated reference without resulting in a change in the basic function of the subject matter at issue.

Having described the invention in detail and by reference to specific embodiments thereof, it will be apparent that modification and variations are possible without departing from the scope of the invention defined in the appended claims. More specifically, although some aspects of the present invention are identified herein as preferred or particularly advantageous, it is contemplated that the present invention is not necessarily limited to these preferred aspects of the invention.

## Claims

1. An infusion system (10) for pumping fluid into a body of a user, the system comprising
- an infusion pump (12) with a housing (21), the housing comprising a cavity (30) for receiving a fluid storing means (15),
- and an adapter (16) that can be connected to a cartridge (14) containing medication, thereby forming a fluid storing means (15) that can be removably inserted into the cavity (30),
wherein the adapter (16) comprises a cannula or a hollow needle (60) for fluidly connecting the adapter (16) to the cartridge (14) through a sealable septum (46) of the cartridge when the adapter is connected to the cartridge, wherein the adapter (16) comprises an adapter housing (50, 50a, 50b) having an hollow interior (56) for receiving the cartridge (14), and wherein the adapter (16) further comprises connection means (58) to removably connect an infusion set, wherein the connection means is a luer connection or a proprietary connection to the infusion set, and that the infusion system (10) further comprises a cap (18) for holding the fluid storing means (15) in place inside the cavity (30), by coupling the cap (18) to the housing (21) after the fluid storing means having been inserted into the cavity, **characterized in that** the adapter further comprises attaching means in the interior of the adapter housing (50, 50a, 50b) for providing a tight fit between the adapter (16) and the cartridge (14).

2. The system according to claim 1, **characterised in that** the adapter (16) is provided with a torque transfer mechanism (64) for transferring torque from the adapter to the cartridge (14), in order to couple a plunger (34) of the cartridge with a drive unit (32) of the infusion pump (12).

3. The system according to claim 2, **characterised in that** the torque transfer mechanism (64) comprises a plurality of ribs (64) provided in the hollow interior (56) of the adapter housing (50).

4. The system according any of the preceding claims, **characterised by** a cartridge (14) connected to the adapter (16).

5. The system according to any of the preceding claims, **characterised in that** the housing (50) of the adapter (16) comprises a part (50a) that can be removably or fixedly attached into the cavity (30).

6. The system according to any of the preceding claims, **characterised in that**
- the adapter (16) is provided with a spring material to cover the tolerances between the cartridge (14) and the adapter housing (50, 50a, 50b) and / or
- the adapter housing is formed of two parts (50a, 50b), wherein a first part (50a) is provided with a spring or a shock absorbing material to cover the tolerances between the adapter (16) and the pump housing (21) and / or
- the adapter (16) is designed such that it works as a spring or shock absorbing element to cover the tolerances between the cartridge (14) and the infusion pump housing (21), wherein the hollow interior (56) of the adapter (16) is provided with a spring mechanism or shock absorbing material.

7. The system according to any of any of the preceding claims, **characterised in that** the housing (50) of the adapter (16) is formed of two parts (50a, 50b), wherein a first part (50a) is placed inside the cavity (30), and a second part (50b) comprising the cannula (60) and the connecting means (58) can be coupled or mated to the first part (50a) after the cartridge (14) having been connected to the adapter (16).

8. The system according to claim 7, **characterised in that** the first part (50a) and the second part (50b) can be connected by snapping the two parts together.

9. The system according to any of claims 6 to 8, **characterised in that** the adapter (16) further comprises a valve between the connecting means (58) and the cannula (60) such that the valve prevents free flow of the fluid.

10. The system according to any the preceding claims, **characterised in that** the adapter (16) and the cartridge (14) cannot be separated after having been connected.

## Patentansprüche

1. Infusionssystem (10) zum Pumpen von Flüssigkeit in einen Körper eines Benutzers, wobei das System Folgendes umfasst:
- eine Infusionspumpe (12) mit einem Gehäuse (21), wobei das Gehäuse einen Hohlraum (30) zur Aufnahme eines Fluidspeichermittels (15) umfasst,
- und einen Adapter (16), der mit einer Patrone (14) verbunden werden kann, die ein Medikament enthält, wodurch ein Fluidspeichermittel (15) gebildet wird, das entfernbar in den Hohlraum (30) eingesetzt werden kann,
wobei der Adapter (16) eine Kanüle oder eine Hohlnadel (60) zum fluidischen Verbinden des Adapters (16) mit der Patrone (14) durch ein abdichtbares Septum (46) der Patrone aufweist, wenn der Adapter mit der Patrone verbunden ist, wobei der Adapter (16) ein Adaptergehäuse (50, 50a, 50b) mit einem hohlen Innenraum (56) zur Aufnahme der Patrone (14) umfasst, und wobei der Adapter (16) ferner Verbindungsmittel (58) umfasst, um ein Infusionsset entfernbar zu verbinden, wobei das Verbindungsmittel eine Luer-Verbindung oder eine proprietäre Verbindung zum Infusionsset ist und das Infusionssystem (10) ferner eine Kappe (18) zum Halten des Fluidspeichermittels (15) in dem Hohlraum (30) durch Kopplung der Kappe (18) an das Gehäuse (21) nachdem das Fluidspeichermittel in den Hohlraum eingeführt worden ist, aufweist, **dadurch gekennzeichnet, dass** der Adapter ferner Befestigungsmittel in dem Innenraum des Adaptergehäuses (50, 50a, 50b) aufweist, um eine feste Verbindung zwischen dem Adapter (16) und der Patrone (14) bereitzustellen.

2. System nach Anspruch 1, **dadurch gekennzeichnet, dass** der Adapter (16) mit einem Drehmomentübertragungsmechanismus (64) zum Übertragen von Drehmoment vom Adapter auf die Patrone (14) versehen ist, um einen Stößel (34) der Patrone mit einer Antriebseinheit (32) der Infusionspumpe (12) zu koppeln.

3. System nach Anspruch 2, **dadurch gekennzeichnet, dass** der Drehmomentübertragungsmechanismus (64) mehrere Rippen (64) umfasst, die in dem hohlen Innenraum (56) des Adaptergehäuses (50) vorgesehen sind.

4. System nach einem der vorhergehenden Ansprüche, **gekennzeichnet durch** eine Patrone (14), die mit dem Adapter (16) verbunden ist.

5. System nach einem der vorhergehenden Ansprüche, **dadurch gekennzeichnet, dass** das Gehäuse (50) des Adapters (16) einen Teil (50a) umfasst, der entfernbar oder fest in dem Hohlraum (30) angebracht werden kann.

6. System nach einem der vorhergehenden Ansprüche, **dadurch gekennzeichnet, dass**
- der Adapter (16) mit einem Federmaterial versehen ist, um die Toleranzen zwischen der Patrone (14) und dem Adaptergehäuse (50, 50a, 50b) abzudecken, und/oder
- das Adaptergehäuse aus zwei Teilen (50a, 50b) gebildet ist, wobei ein erstes Teil (50a) mit einer Feder oder einem stoßdämpfenden Material versehen ist, um die Toleranzen zwischen Adapter (16) und Pumpengehäuse (21) auszugleichen, und/oder
- der Adapter (16) so ausgebildet ist, dass er als Feder- oder Stoßdämpferelement die Toleranzen zwischen der Patrone (14) und dem Infusionspumpengehäuse (21) abdeckt, wobei der hohle Innenraum (56) des Adapters (16) mit einem Federmechanismus oder einem stoßdämpfenden Material versehen ist.

7. System nach einem der vorhergehenden Ansprüche, **dadurch gekennzeichnet, dass** das Gehäuse (50) des Adapters (16) aus zwei Teilen (50a, 50b) gebildet ist,
wobei ein erster Teil (50a) im Inneren des Hohlraums (30) angeordnet ist und ein zweiter Teil (50b), der die Kanüle (60) und das Verbindungsmittel (58) umfasst, mit dem ersten Teil (50a) gekoppelt oder verbunden werden kann, nachdem die Patrone (14) an den Adapter (16) angeschlossen wurde.

8. System nach Anspruch 7, **dadurch gekennzeichnet, dass** der erste Teil (50a) und der zweite Teil (50b) durch Zusammenschnappen der beiden Teile verbindbar sind.

9. System nach einem der Ansprüche 6 bis 8, **dadurch gekennzeichnet, dass** der Adapter (16) ferner ein Ventil zwischen den Verbindungsmitteln (58) und der Kanüle (60) umfasst, so dass das Ventil einen freien Fluss des Fluids verhindert.

10. System nach einem der vorhergehenden Ansprüche, **dadurch gekennzeichnet, dass** der Adapter (16) und die Patrone (14) nach dem Verbinden nicht trennbar sind.

## Revendications

1. Système de perfusion (10) pour le pompage de fluide dans le corps d'un utilisateur, ce système comprenant
- une pompe de perfusion (12) comportant un boîtier (21), ce boîtier comprenant une cavité (30) destinée à recevoir un moyen de stockage de fluide (15),
- et un adaptateur (16) qui peut être connecté à une cartouche (14) contenant du médicament, en formant ainsi un moyen de stockage de fluide (15) qui peut être inséré de manière amovible dans la cavité (30),
dans lequel l'adaptateur (16) comprend une canule ou une aiguille creuse (60) destinée à connecter au niveau fluidique l'adaptateur (16) à la cartouche (14) par l'intermédiaire d'un septum colmatable (46) de la cartouche lorsque l'adaptateur est connecté à la cartouche,
l'adaptateur (16) comprend un boîtier d'adaptateur (50, 50a, 50b) comportant un intérieur creux (56) destiné à recevoir la cartouche (14), et l'adaptateur (16) comprend en outre un moyen de connexion (58) destiné à connecter de manière amovible un ensemble de perfusion, le moyen de connexion est un connecteur Luer ou un connecteur propriétaire de l'ensemble de perfusion, et le système de perfusion (10) comprend en outre un capuchon (18) pour maintenir le moyen de stockage de fluide (15) en place dans la cavité (30) en couplant le capuchon (18) au boîtier (21) une fois que le moyen de stockage de fluide a été inséré dans la cavité, **caractérisé en ce que** l'adaptateur comprend en outre un moyen de fixation dans l'intérieur du boîtier d'adaptateur (50, 50a, 50b) pour établir un ajustement serré entre l'adaptateur (16) et la cartouche (14).

2. Système selon la revendication 1, **caractérisé en ce que** l'adaptateur (16) est pourvu d'un mécanisme de transfert de couple (64) pour transférer du couple de l'adaptateur vers la cartouche (14) afin de coupler un plongeur (34) de la cartouche avec une unité d'entraînement (32) de la pompe de perfusion (12).

3. Système selon la revendication 2, **caractérisé en ce que** le mécanisme de transfert de couple (64) comprend une pluralité de nervures (64) prévues dans l'intérieur creux (56) du boîtier d'adaptateur (50).

4. Système selon l'une quelconque des revendications précédentes, **caractérisé par** une cartouche (14) connectée à l'adaptateur (16).

5. Système selon l'une quelconque des revendications précédentes, **caractérisé en ce que** le boîtier (50) de l'adaptateur (16) comprend une pièce (50a) qui peut être fixée de manière amovible ou fixe dans la cavité (30) .

6. Système selon l'une quelconque des revendications précédentes, **caractérisé en ce que**
- l'adaptateur (16) est pourvu d'un matériau à ressort pour couvrir les tolérances entre la cartouche (14) et le boîtier d'adaptateur (50, 50a, 50b) et/ou
- le boîtier de l'adaptateur est formé de deux pièces (50a, 50b), une première pièce (50a) étant pourvue d'un ressort ou d'un matériau absorbant les chocs pour couvrir les tolérances entre l'adaptateur (16) et le boîtier de pompe (21) et/ou
- l'adaptateur (16) est conçu pour fonctionner en tant que ressort ou qu'élément absorbant les chocs pour couvrir les tolérances entre la cartouche (14) et le boîtier de la pompe de perfusion (21), l'intérieur creux (56) de l'adaptateur (16) étant pourvu d'un mécanisme à ressort ou d'un matériau absorbant les chocs.

7. Système selon l'une quelconque des revendications précédentes, **caractérisé en ce que** le boîtier (50) de l'adaptateur (16) est formé de deux pièces (50a, 50b), une première pièce (50a) étant placée dans la cavité (30), et une seconde pièce (50b) comprenant la canule (60) et le moyen de connexion (58) pouvant être couplé ou adapté à la première pièce (50a) une fois que la cartouche (14) a été connectée à l'adaptateur (16).

8. Système selon la revendication 7, **caractérisé en ce que** la première pièce (50a) et la seconde pièce (50b) peuvent être connectées en enclenchant les deux pièces ensemble.

9. Système selon l'une quelconque des revendications 6 à 8, **caractérisé en ce que** l'adaptateur (16) comprend en outre une vanne entre le moyen de connexion (58) et la canule (60) de manière à ce que la vanne empêche un écoulement libre du fluide.

10. Système selon l'une quelconque des revendications précédentes, **caractérisé en ce que** l'adaptateur (16) et la cartouche (14) ne peuvent pas être séparés après avoir été connectés.
